# EUROPEAN PATENT APPLICATION

(11) **EP 3 900 695 A1**
(43) Date of publication of application: **27.10.2021**
(21) Application number: 20170330.3
(22) Date of filing: 20.04.2020
(51) Int. Cl.: A61K 8/02, A61K 8/87, A61Q 19/00

(54) **PEEL-OFF MASK COMPOSITION**

(71) Applicant: Covestro Deutschland AG, 51373 Leverkusen (DE)
(72) Inventor: The designation of the inventor has not yet been filed
(74) Representative: Levpat

(57) **Abstract**

The invention relates to a peel-off mask composition, in particular to a dry peel-off mask composition, a method for preparing the same, and a method for applying and using the same. The peel-off mask composition comprises the following components: a) at least an aqueous polyurethane dispersion comprising a polyurethane and water; b) at least a liquid inverse emulsion thickener; c) at least a non-silicone oil based fat or oil, and d) optionally an additive; wherein the amount of the polyurethane is 8 wt% to 29 wt%, the amount of the liquid inverse emulsion thickener is 0.8 wt% to 6 wt%, the amount of the non-silicone oil based fat or oil is 1 wt% to 8 wt%, the above weight proportions being all based on the amount of the mask composition being 100 wt%.

## Description

### Technical field

The invention relates to a peel-off mask composition, a method for preparing the same, and a method for applying and using the same.

### Prior art

There are various types of masks, for example, patch masks, washable masks (including mud-like washable masks, cream-like washable masks, and gel-like washable masks), leave-on sleep masks, and peel-off masks (peelable masks).

A liquid of peel-off masks in the form of lotion, cream or spray is applied to the face uniformly; after 15-20 minutes, the mask is peeled from the face by hand. Peel-off masks can clean pores and remove excess grease. They may be classified to two types according to the usage ways: dry peel-off masks and wet peel-off masks. Dry peek-off masks refer to masks that can be completely removed directly after drying. Wet peel-off masks refer to masks that can be removed completely only after being re-wetted with water. Due to the convenience of dry peel-off masks, they are very popular for consumers.

In order to ensure that the dry peel-off masks can form a continuous film on the skin surface in a short time, and that the film does not break during the peeling process, the film-forming agent in the dry peel-off masks is extremely important. The films should have good elasticity (i.e., elongation at break), good tensile strength, and moderate adhesion between the film and the skin. When a mask comprises polyvinyl alcohols or polyvinyl pyrrolidones as a film-forming agent, users have a strongly tight feeling on the skin after the masks containing said film-forming agent are dry, and it is very painful on the skin during the peeling process of the film due to the strong adhesion between the film and the skin. Thus, these masks show poor consumer experience. The film formed by a composition comprising an acrylic film-forming agent is too hard, breaks easily, and cannot be completely removed from the skin even if it is wetted with water. A mask comprising polyurethanes as a film-forming agent has good elasticity, and can be completely removed from the skin after the film formed by the composition is re-wetted. The users feel no pain.

For example, CN104352359A discloses a peel-off mask based on an aqueous polyurethane dispersion, and mentions that it needs to be wetted with water to be peeled off. CN104069052B discloses an elastic memory mask that mimics the effect of an eggshell film, which must also be wetted with water before being peeled off. CN107823099A discloses an acne marks-removing mask, which comprises a polyurethane film-forming agent in combination with a mask cloth and belongs to a patch-type mask. CN108135805A discloses a beauty mask, which comprises a water-insoluble matrix with impregnated whitening particles and a film-forming polymer and has an immediate brightening effect, and also belongs to a mask combination of a patch mask and a polymer film-forming agent.

It can be seen that none of the above masks can achieve a dry peel-off effect. It is desired to develop a dry peel-off mask that can be completely removed from the skin surface without being wetted with water, and can make the users painless.

### Detailed description of the invention

The objects of the present invention are to provide a peel-off mask composition, especially a dry peel-off mask composition, a method for preparing the same, and a method for applying and using the same.

A peel-off mask composition of the present invention comprises the following components:
a) at least an aqueous polyurethane dispersion comprising a polyurethane and water;
b) at least a liquid inverse emulsion thickener;
c) at least a non-silicone oil based fat or oil, and
d) optionally an additive;
wherein the amount of the polyurethane is 8 wt% to 29 wt%, the amount of the liquid inverse emulsion thickener is 0.8 wt% to 6 wt%, the amount of the non-silicone oil based fat or oil is 1 wt% to 8 wt%, the above weight proportions being all based on the amount of the mask composition being 100 wt%.

According to one aspect of the present invention, a method for preparing a peel-off mask composition of the present invention is provided, including the following steps: mixing component a) aqueous polyurethane dispersion, component b) liquid inverse emulsion thickener and component c) non-silicone oil based fat or oil, and component d) optionally additives to obtain the mask composition.

According to yet another aspect of the present invention, use of the peel-off mask composition of the present invention for applying on the skin to take care of the skin is provided.

According to yet another aspect of the present invention, a method for taking care of the skin is provided, including applying the peel-off mask composition of the present invention on the skin surface;
drying to form a film; and
peeling it from the skin.

The peel-off mask composition of the present invention can form a tough, continuous and elastic film, and the adhesion between the film and the skin is moderate. It is not necessary to wet the film before peeling it from the skin surface. The peeling process makes the users painless and without obvious greasy feeling. In addition, the peel-off mask composition of the present invention can quickly dry to form a film after being applied to the skin, which greatly reduces the users' waiting time for the mask to dry.

### Embodiments

The present invention provides a peel-off mask composition comprising the following components: a) at least an aqueous polyurethane dispersion comprising a polyurethane and water; b) at least a liquid inverse emulsion thickener; c) at least a non-silicone oil based fat or oil, and d) optionally an additive; wherein the amount of the polyurethane is 8 wt% to 29 wt%, the amount of the liquid inverse emulsion thickener is 0.8 wt% to 6 wt%, the amount of the non-silicone oil based fat or oil is 1 wt% to 8 wt%, the above weight proportions being all based on the amount of the mask composition being 100 wt%. The present invention also provides a method for preparing the same, and a method for applying and using the same.

The mask composition is preferably a dry peel-off mask.

### Component a) aqueous polyurethane dispersion

Within the scope of the present invention, the term "water-insoluble and water-nondispersible isocyanate-functionalized polyurethane prepolymer" means that the prepolymer has a solubility in water at 23 °C of less than 10 g/liter, preferably less than 5 g/liter, and the prepolymer is unable to produce a sedimentation stable dispersion in water (especially deionized water) at 23 °C. In other words, the prepolymer precipitates out in experiments in which it is dispersed in water.

The aqueous polyurethane dispersion may be added to the mask composition in the form of a solid or a dispersion, and is preferably added to the mask composition in the form of a dispersion, that is, added to the mask composition in the form of an aqueous polyurethane dispersion. The polyurethane added to the mask composition in a solid form may be dispersed in water of the mask composition to form an aqueous polyurethane dispersion.

For a composition in the form of a dispersion, the weight of the polyurethane (i.e. the weight of the effective components of the aqueous polyurethane dispersion or the weight of the solid components of the aqueous polyurethane dispersion) = the weight of the dispersion × the content of the solid components of the dispersion. For a solid substance containing crystal water, the weight of the polyurethane (i.e. the weight of the effective components or the weight of the solid components) = the weight of the substance - the weight of the crystal water.

The polyurethane is preferably an anionic polyurethane.

The anionic polyurethane is preferably obtained by the reaction of a system comprising a1) at least a water-insoluble and water-nondispersible isocyanate- functionalized polyurethane prepolymer and a2) at least an isocyanate-reactive compound.

The anionic polyurethane comprises preferably at least a sulfonic acid group and/or sulfonate group, and most preferably at least a sodium sulfonate group.

The anionic polyurethane may be linear or branched, and is most preferably linear.

The number average molecular weight of the anionic polyurethane may be 100,000 g/mol to 500,000g/mol, further preferably 120,000 g/mol to 400,000 g/mol, and most preferably 150,000 g/mol to 400,000 g/mol.

### a1) Water-insoluble and water-nondispersible isocyanate-functionalized polyurethane prepolymer

The water-insoluble and water-nondispersible isocyanate-functionalized polyurethane prepolymer has preferably a terminal isocyanate group, that is, the isocyanate group is located at the end of the prepolymer chain, and most preferably, all the chain ends of the polyurethane prepolymer have isocyanate group.

The water-insoluble and water-nondispersible isocyanate-functionalized polyurethane prepolymer is substantially free of ionic groups and/or ionogenic groups, that is, the content of the ionic groups and/or ionogenic groups is preferably less than 15 mg equivalent/100 g of polyurethane prepolymer, further preferably less than 5 mg equivalent/100 g of polyurethane prepolymer, more preferably less than 1 mg equivalent/100 g of polyurethane prepolymer, most preferably less 0.1 mg equivalent/100 g of polyurethane prepolymer. The water here means deionized water without any surfactant added.

The water-insoluble and water-nondispersible isocyanate-functionalized polyurethane prepolymer can be prepared by the reaction of a system comprising a polyol, an organic polyisocyanate, optionally a hydroxyl-functional compound having a number average molecular weight of 62 to 399 g/mol, and optionally a non-ionic hydrophilizing reagent.

The polyol is preferably one or more selected from polyether polyols, polycarbonate polyols, polyether polycarbonate polyols, and polyester polyols, further preferably a polyol having a linear structure, and still more preferably one or more selected from polytetramethylene glycol polyethers and polycarbonate polyols, most preferably polytetramethylene glycol polyethers.

The number average molecular weight of the polyol is preferably 400 g/mol to 6000 g/mol, and most preferably 600 g/mol to 3000 g/mol.

The hydroxyl functionality of the polyol is preferably 1.5 to 6, more preferably 1.8 to 3, and most preferably 1.9 to 2.1.

The isocyanate group (NCO) functionality of the organic polyisocyanate is preferably not less than 2.

The organic polyisocyanate is preferably one or more selected from aliphatic polyisocyanate, aromatic polyisocyanate, and alicyclic polyisocyanate, further preferably one or more selected from 1,4-butylene diisocyanate, 1,6-hexamethylene diisocyanate (HDI), isophorone diisocyanate (IPDI), 2,2,4- and/or 2,4,4-trimethylhexamethylene diisocyanate, bis(4,4'-isocyanatocyclohexyl) methane isomers or mixtures of these isomers, 1,4-cyclohexylene diisocyanate, 4-isocyanatomethyl-1,8-octane diisocyanate (nonane triisocyanate), 1,4-benzene diisocyanate, 2,4- and/or 2,6-toluene diisocyanate, 1,5-naphthylene diisocyanate, 2,2'- and/or 2,4'- and/or 4,4'-diphenylmethane diisocyanate, 1,3- and/or 1,4-bis(2-isocyanatoprop-2-yl) benzene (TMXDI), 1,3-bis(isocyanatomethyl) benzene (XDI) and C1-C8-alkyl 2,6-diisocyanatohexanoate (lysine diisocyanate), most preferably one or more selected from hexamethylene diisocyanate and isophorone diisocyanate.

The organic polyisocyanate may also be a polyisocyanate containing a hetero atom in the groups bonded to isocyanate groups, for example, a polyisocyanate obtained by modification using uretdione, isocyanurate, urethane, allophanate, biuret, carbodiimide, iminooxadiazinedione and/or oxadiazinetrione structure.

The hydroxy-functional compound having a molecular weight of 62 to 399 mol/g is preferably one or more selected from non-polymeric polyols having no more than 20 carbon atoms, ester diols, and monofunctional hydroxy compounds.

The non-polymeric polyol having no more than 20 carbon atoms is preferably one or more selected from ethylene glycol, diethylene glycol, triethylene glycol, 1,2-propanediol, 1,3-propanediol, 1,4-butanediol, 1,3-butanediol, cyclohexanediol, 1,4-cyclohexane dimethanol, 1,6-hexanediol, neopentyl glycol, hydroquinone dihydroxyethyl ether , bisphenol A (2,2-bis(4-hydroxyphenyl) propane), hydrogenated bisphenol A (2,2-bis(4-hydroxycyclohexyl) propane), trimethylol propane, trimethylol ethane, glycerol and pentaerythritol.

The ester diol is preferably one or more selected from α-hydroxybutyl ε-hydroxyhexanoate, ω-hydroxyhexyl γ-hydroxybutyrate, (β-hydroxyethyl) adipate and di(β-hydroxyethyl) terephthalate.

The monofunctional hydroxy compound is preferably one or more selected from ethanol, n-butanol, ethylene glycol monobutyl ether, diethylene glycol monomethyl ether, diethylene glycol monobutyl ether, propylene glycol monomethyl ether, dipropylene glycol monomethyl ether, tripropylene glycol monomethyl ether, dipropylene glycol monopropyl ether, propylene glycol monobutyl ether, dipropylene glycol monobutyl ether, tripropylene glycol monobutyl ether, 2-ethyl hexanol, 1-octanol , 1-dodecanol, and 1-hexadecanol.

The non-ionic hydrophilicizing agent is preferably one or more selected from polyoxyalkylene ethers containing a hydroxyl group, an amino group and a thiol group.

The non-ionic hydrophilizing agent is preferably a monohydroxy-functionalized polyoxyalkylene polyether having 5 to 70, preferably 7 to 55 ethylene oxide units per molecule. The polyoxyalkylene polyether can be obtained by alkoxylation of a suitable starter molecule according to known methods.

The monohydroxy-functionalized polyoxyalkylene polyether is preferably pure polyoxyethylene polyether or mixed polyoxyalkylene polyethers. The polyoxyethylene polyether contains preferably not less than 30 mol%, and most preferably not less than 40 mol% of ethylene oxide units, based on the alkylene oxide units being 100 mol%.

### a2) Isocyanate-reactive compound

The isocyanate-reactive compound is preferably one or more selected from amino-functional compounds and hydroxy-functional compounds.

The amino-functional compound is preferably one or more selected from primary amines, secondary amines and diamines, most preferably diamines.

The amino-functional compound is preferably an amino-functional compound without any ionic group or ionogenic group, or an amino-functional compound with an ionic group or ionogenic group.

The amino-functional compound without any ionic group or ionogenic group is preferably one or more selected from organic diamines, polyamines, compounds containing a primary and secondary amino group, compounds containing a (primary or secondary) amino group and a hydroxyl groups, and monofunctional isocyanate-reactive amine compounds.

The organic diamine is preferably one or more selected from 1,2-ethylene diamine, 1,2- and 1,3-diaminopropane, 1,4-diaminobutane, 1,6-diaminohexane, isophorone diamine, isomer mixtures of 2,2,4- and 2,4,4-trimethylhexamethylene diamine, 2-methylpentamethylene diamine, 4,4-diaminodicyclohexylmethane, hydrazine hydrate and dimethylethylene diamine.

The polyamine is preferably diethylene triamine.

The compound containing a primary and secondary amino group is preferably one or more selected from diethanol amine, 3-amino-1-methylaminopropane, 3-amino-1-ethylaminopropane, 3-amino-1-cyclohexylaminopropane and 3-amino-1-methylaminobutane.

The compound containing a (primary or secondary) amino group and a hydroxyl groups, the alcohol amine is preferably one or more selected from N-aminoethyl ethanolamine, ethanolamine, 3-aminopropanol, and neopentanol amine.

The monofunctional isocyanate-reactive amine compound is preferably one or more selected from methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine and suitable substituted derivatives thereof.

The amino-functional compound without any ionic group or ionogenic group is most preferably a diamine without any ionic group or ionogenic group.

The amino-functional compound with an ionic group or ionogenic group is preferably an anionic hydrophilized compound.

The anionic hydrophilized compound is preferably one or more selected from 2-(2-aminoethylamino)ethanesulfonic acid, ethylenediamine-propyl or butyl-sulfonic acid, 1,2- or 1,3-propanediamine-β-ethylsulfonic acid and taurine and salts thereof, further preferably those containing a sulfonate group as an ionic group and two amino groups, most preferably one or more of the following compounds: 2-(2-aminoethylamino)ethylsulfonate and 1,3-propanediamine-β-ethylsulfonate.

The hydroxy-functional compound is preferably a polymer polyol, and further preferably one or more selected from polyester polyols, polyacrylate polyols, polyurethane polyols, polycarbonate polyols, polyether polyols, polyester polyacrylate polyols, polyurethane polyacrylate polyols, polyurethane polyester polyols, polyurethane polyether polyols, polyurethane polycarbonate polyols, and polyester polycarbonate polyols, and more preferably one or more selected from polytetramethylene glycol polyethers and polycarbonate polyols, most preferably polytetramethylene glycol polyethers.

The aqueous anionic polyurethane dispersion is preferably one or more selected from polyurethane-34, polyurethane-32, polyurethane-35, polyurethane-48, polyurethane-11 and polyurethane-2, the above names all being according to the International Cosmetic Ingredient Dictionary and Handbook (INCI); most preferably one or more selected from Baycusan®C1000/1, Baycusan®C1001/1, Baycusan®C1003/1, Baycusan®C1004/1, Baycusan®C1008/1, Baycusan®C1010, Carfil®9221, Carfil® UV35, Carfil® 9235NP and Carfil® ST11.

### Preparation of aqueous anionic polyurethane dispersions

The method for preparing aqueous anionic polyurethane dispersions includes the following steps:
preparing a1) a water-insoluble and water-nondispersible isocyanate-functionalized polyurethane prepolymer;
reacting a1) the polyurethane prepolymer with a2) an isocyanate-reactive compound; and dispersing the anionic polyurethane in water before, during or after the reaction.

The anionic groups contained in the aqueous anionic polyurethane dispersion are introduced by the amino-functional compounds with an ionic group or ionogenic group as the isocyanate-reactive compounds.

The preparation of aqueous anionic polyurethane dispersions can be performed in a homogeneous phase in one or more stages, or partly in a dispersed phase for a multi-stage reaction. The method for preparing the aqueous anionic polyurethane dispersions may be the prepolymer mixing method, the acetone method, or the melt dispersing method, and is preferably the acetone method.

The equivalent ratio of the isocyanate groups to the isocyanate-reactive groups is preferably 1.05 to 3.5, more preferably 1.1 to 3.0, and most preferably 1.1 to 2.5.

The particle size of the anionic polyurethanes is preferably less than 750 nm, and most preferably less than 500 nm. The particle size of the present invention is measured using a Malvern Zetasizer 1000 from Malver after diluting the anionic polyurethane with deionized water.

The solid content of the aqueous anionic polyurethane dispersion is preferably 5 wt% to 50 wt%, and most preferably 10 wt% to 15 wt%, based on the amount of the aqueous anionic polyurethane dispersion being 100 wt%. The solid content is calculated after heating the weighed sample to a constant weight at 125 °C and reweighing the sample under the constant weight.

### Component b) liquid inverse emulsion thickener

The liquid inverse emulsion thickener of the present invention can also be called a liquid phase inverse emulsion thickener, a liquid (phase) inverse emulsion thickener, or a hydro swelling droplets (HSD) thickener.

The liquid inverse emulsion thickener of the present invention is preferably a compounded product of a system comprising at least one of alkanes and isoalkanes, a hydrophobically modified acrylic copolymer, and a nonionic emulsifier of oil-in-water type. Specifically, a compound is firstly formed from at least one of alkanes and isoalkanes, a hydrophobically modified acrylic copolymer, and a nonionic emulsifier of oil-in-water type. Then the compound is added to water to form a liquid inverse emulsion thickener. The hydrophobically modified acrylic copolymer is preferably a pre-neutralized hydrophobically modified acrylic copolymer, more preferably a pre-neutralized hydrophobically modified acrylic copolymer in a spiral form, and most preferably one or more selected from polyacrylamide, sodium acryloyldimethyltaurate copolymer and ammonium acryloyldimethyltaurate copolymer. The continuous phase of the liquid inverse emulsion thickener is the at least one of alkanes and isoalkanes, and the internal phase is the hydrophobically modified acrylic copolymer, especially the pre-neutralized hydrophobically modified acrylic copolymer in a spiral form. The function of the nonionic emulsifiers of oil-in-water type is to facilitate the phase transition of the hydrophobically modified acrylic copolymer. The compound is initially in the form of an inverse emulsion of oil-in-water type. The inverse emulsion undergoes phase transition when the compound is added to water. The chains of the pre-neutralized polymer stretch freely in the external phase, expand and swell, resulting in a viscous and stable gel, which is the liquid inverse emulsion thickener of the present invention.

The liquid inverse emulsion thickener is preferably one or more selected from Simugel™EG, Simugel™EG QD, Simugel™EPG QD, Simugel™FL, Simugel™ NS, Simugel™ EPG, Simugel™ SMS 88, Simugel™ INS 100, Sepiplus™ S, Sepiplus™ 265, Sepiplus™ 400, Sepigel™ 305, aquagel 35, aquagel 45, aquagel 55, aquagel 65, novemer™ ec-1 polymer, novemer™ ec-2 polymer and novemer™ ec-2cc polymer.

The amount of component b) liquid inverse emulsion thickener is preferably 1 wt% to 3 wt%, based on the amount of the peel-off mask composition being 100 wt%. When the liquid inverse emulsion thickener is in the form of a dispersion or a substance containing crystal water, the amount of the liquid inverse emulsion thickener refers to the amount of the effective or solid components.

### Component c) non-silicone oil based fat or oil

The non-silicone oil based fat or oil is preferably one or more selected from non-silicone oil based vegetable oils, non-silicone oil based animal fats, non-silicone oil based mineral oils and non-silicone oil based synthetic fats or oils; most preferably one or more selected from caprylic/capric triglyceride, macadamia oil and isododecane.

The amount of the non-silicone oil based fat or oil is preferably 3 wt% to 5 wt%, based on the amount of the peel-off mask composition being 100 wt%.

### Component e) additives

The additives of the present invention are those that are physically and chemically compatible with components in the mask composition of the present invention.

The additive is preferably one or more selected from thickeners different from component b), fats or oils different from component c), emulsifiers, preservatives and humectants.

The thickener different from component b) is preferably one or more selected from acrylics, celluloses, diatomaceous earth, Carbopol, starch, gelatin, sodium alginate, casein, guar gum, chitosan, gum arabic, xanthan gum, natural lanolin and agar.

The amount of the thickener different from component b) is preferably 0 to 10 wt%, based on the amount of the mask composition being 100 wt%.

The fat or oil different from component c) is preferably one or more selected from vegetable oils, animal fats, mineral oils, and synthetic fats or oils, and most preferably one or more selected from olive oil, coconut oil, castor oil, cottonseed oil, soybean oil, sesame oil, almond oil, peanut oil, corn oil, rice bran oil, tea seed oil, sea buckthorn oil, avocado oil, candlenut oil, european nut oil, walnut oil, cocoa oil, mink oil, yolk oil, lanolin oil, lecithin, squalane, lanolin derivatives, polysiloxanes, fatty acids, fatty alcohols, fatty acid esters, and vaseline.

The amount of the fat or oil different from component c) is preferably 0 to 10 wt%, based on the amount of the mask composition being 100 wt%.

The emulsifier is preferably one or more selected from carboxylates, sulfates, sulfonates, amine derivatives, alkyl ethers, stearyl esters, polyoxyethylene ethers, and polyoxypropylene ethers.

The amount of the emulsifier is preferably 0 to 10 wt%, based on the amount of the mask composition being 100 wt%.

The preservative is preferably one or more selected from alcohol preservatives, donors of formaldehyde and preservatives of aldehyde derivatives, preservatives of benzoic acid and derivatives thereof, sodium hydroxymethylglycine, hydroxyacetophenone, and preservative compounds, more preferably compounds of phenoxyethanol and ethylhexyl glycerol, and/or compounds of methyl propylene glycol, caprylyl glycol, and phenylpropanol, most preferably Dermosoft OMP.

The amount of the preservative is preferably 0.5 wt% to 2 wt%, based on the amount of the mask composition being 100 wt%.

The humectant is preferably one or more selected from polyols, amides, lactic acids and sodium lactates, sodium pyrrolidonecarboxylate, glucose esters, collagens, chitin derivatives, and chitosans, and most preferably polyols.

The amount of the humectant is preferably 0 to 20 wt%, based on the amount of the mask composition being 100 wt%.

### Component e) cosmetically acceptable medium

The mask composition may further comprises a cosmetically acceptable medium. The cosmetically acceptable medium can be selected based on other ingredients in the mask composition.

The cosmetically acceptable medium is preferably one or more selected from water and aqueous solutions of lower alkanols, more preferably water, and most preferably deionized water.

The aqueous solution of a lower alkanol comprises preferably a monohydric alcohol having 1 to 6 carbon atoms, and most preferably one or more selected from ethanol and isopropanol.

The amount of the cosmetically acceptable medium may be an amount well known to those skilled in the art and the amount of the cosmetically acceptable medium is preferably 20 to 90 wt%, more preferably 30 to 90 wt%, and most preferably 40 to 80 wt%, based on the amount of the mask composition being 100 wt%

### Method for preparing mask compositions

Preferably, the method for preparing a mask composition of the present invention includes the following steps: mixing component a) aqueous polyurethane dispersion, component b) liquid inverse emulsion thickener, component c) non-silicone oil based fat or oil, component d) optionally additives and components e) optionally a cosmetically acceptable medium to obtain the mask composition.

Most preferably, the method for preparing a mask composition of the present invention includes the following steps:
i) mixing the thickener different from component b), component d) optionally additives, and optionally water;
ii) adding components b) and component c);
iii) adding component a) and optionally a preservative, and optionally adding a neutralizing agent to adjust the pH value to 6.5 to 7.0, to obtain the mask composition.

The step i) may be heating operation. The substance obtained in step i) is preferably cooled to 40 °C to 50 °C before step ii).

In step ii), component c) is added at room temperature, if component c) non-silicone oil based fat or oil is liquid at room temperature. If component c) non-silicone oil based fat or oil is solid at room temperature, the non-silicone oil based fat or oil is firstly heated to 70 °C to 90 °C to melt and then mixed with component b). The mixture is stirred well at 70 °C to 90 °C, and then cooled to 45 °C or lower.

### Mask composition

The mask composition may be spray-like, gel-like, cream-like or emulsion-like.

### Examples

Unless defined otherwise, all technical and scientific terms used herein have the same meaning as commonly understood by one of ordinary skill in the art. When the definitions of the terms in this specification conflict with the meanings commonly understood by those skilled in the art, the definitions described herein shall apply.

Unless otherwise stated, all numerical values of the amounts of components, reaction conditions, and the like used in the specification and claims are understood to be modified by the term "about". Therefore, unless indicated to the contrary, the numerical values set forth herein are approximations that can vary depending on the required performance required to be obtained.

As used herein, "and/or" means one or all of the elements mentioned.

As used herein, "including" and "comprising" encompass situations where there are only the mentioned elements, as well as situations where there are other elements in addition to the mentioned elements.

All percentages in the present invention are weight percentages, and are based on the amount of the cosmetic composition of the present invention being 100 wt%, unless otherwise stated.

The molecular weight of the present invention is a number average molecular weight, unless otherwise stated. The number average molecular weight was determined by gel permeation chromatography (GPC) method according to GB/T 21863-2008 "Gel permeation chromatography (GPC) - Tetrahydrofuran as elution solvent".

The analytical measurements of the present invention were all performed at 23 ± 2 °C, unless otherwise stated.

The solid content of the aqueous polyurethane dispersion was measured using a HS153 moisture analyzer from Mettler Toledo according to DIN-EN ISO 3251.

The isocyanate group (NCO) content was determined by volume according to DIN-EN ISO 11909, the measured data referring to both free and potentially free NCO groups.

Isocyanate functionality was measured according to GPC.

The pH value was determined according to GB/T 13531.1-2008.

### Raw materials and reagents

Carbopol Ultrez 20: acrylic acid/C10-30 alkyl acrylate crosspolymer (INCI name) with a solid content of 100 wt%, purchased from Lubrizol.

Simugel INS 100: hydroxyethyl acrylate/sodium acryloyldimethyltaurate copolymer and isohexadecane and polysorbate-60 (INCI name) with a solid content of 100 wt%, purchased from Seppic.

Sepigel 305: polyacrylamide and C13-14 isoparaffin and laureth-7 (INCI name) with a solid content of 100%, purchased from Seppic.

Semugel EG: sodium acrylate/sodium acryloyldimethyltaurate copolymer and isohexadecane and polysorbate-80 (INCI name) with a solid content of 100 wt%, purchased from Seppic.

Sepiplus 400: polyacrylate-13 and polyisobutylene and polysorbate-20 (INCI name) with a solid content of 100 wt%, purchased from Seppic.

Baycusan® C1004/1: aqueous anionic polyurethane dispersion with a solid content of 41 wt% and INCI name of polyurethane-35, available from Covestro Polymers (China) Co., Ltd.

Baycusan® C1008/1: aqueous anionic polyurethane dispersion with a solid content of 30 wt% and INCI name of polyurethane-48, available from Covestro Polymers (China) Co., Ltd.

Carfil ST11: polyurethane-35 and polyurethane-11 (INCI name) with a solid content of 45 wt%, available from Wanhua Chemical Group Co., Ltd.

Carfil 9221: polyurethane-35 (INCI name) with a solid content of 40 wt%, available from Wanhua Chemical Group Co., Ltd.

Baycusan® C1001/1: aqueous anionic polyurethane dispersion with a solid content of 32 wt% and INCI name of polyurethane-34, available from Covestro Polymers (China) Co., Ltd.

Baycusan® C1004/1: aqueous anionic polyurethane dispersion with a solid content of 41 wt% and INCI name of polyurethane-35, available from Covestro Polymers (China) Co., Ltd.

Euxyl® PE 9010: phenoxyethanol and ethylhexyl glycerol (INCI name) with a solid content of 100 wt%, purchased from Schülke & Mayr GmbH.

DOWSIL™ SH 245: cyclopentasiloxane (INCI name) with a solid content of 100 wt%, purchased from Dow Chemical.

DOWSIL™ SH 556: phenyl trimethicone (INCI name) with a solid content of 100 wt%, purchased from Dow Chemical.

Triethanol amine: concentration of 99%, purchased from Shanghai Lingfeng Chemical Reagent Co., Ltd

### Preparation of mask compositions

According to the composition listed in the table, Carbopol® Ultrez 20 was added to a beaker pre-filled with deionized water, and stirred well until the solution in the beaker became completely transparent. Component b) was added to the mixture while stirring und dispersed homogeneously. Then component c) was added while stirring. After 5 minutes of stirring, component a) and component d) were added und stirred continuously until the mixture became homogeneous.

### Performance Testing

### Adhesion of the mask composition on the skin surface, users' pain feeling, and the residual amount of fat or oil on the skin

A 4 cm * 2 cm square was drawn on the inside of the arm. A 0.2 g sample was weighed and applied uniformly on the square. After the sample was completely dry and formed into a film, the film was peeled off carefully in one piece. The adhesion of each sample to the skin was recorded and scored. The score was 1 to 5 in order from low to high. Score 1 means that the adhesion is strong and the film cannot be pulled up. Score 5 means that there is no adhesion and the film can be pulled up easily. When each sample was pulled up and peeled off, the users' pain feeling was recorded and scored. The score was 1 to 5 in order from low to high. Score 1 means a strong pain feeling, and score 5 means no pain feeling. The residual amount of fat or oil on the skin was recorded and scored by the users after the sample was peeled off. The score was 1 to 5 in order from low to high. Score 1 means the residual amount of fat or oil is large. Score 5 means no significant fat or oil residue. The residual fat or oil on the skin may leave a greasy feeling and reduce the comfort. A mask composition is considered to have a good dry peel-off effect and an unobvious greasy feeling, which is qualified, when the score for adhesion is greater than or equal to 4, the score for pain feeling is greater than or equal to 4, and the score for residue amount of fat or oil is greater than or equal to 4.

### Elongation length

Three 4 cm * 2 cm squares were drawn on the inside of the arm. Three 0.2 g samples were applied uniformly on the squares. After the samples were completely dried and formed into a film, the films were carefully peeled off in one piece. The peeled films were elongated to break, and the length at the break was measured and recorded as the elongation length. The elongation length was scored with 1 to 5 in order from low to high. Score 4 and above mean that the samples are qualified. A elongation length of 0 to 12 cm is recorded as score 1. A elongation length of more than 12 cm and no more than 20 cm is recorded as score 2. A elongation length of more than 20 cm and no more than 28 cm is recorded as score 3. A elongation length of more than 28 cm and no more than 36 cm is recorded as score 4. A elongation length of more than 36 cm is recorded as score 5. A score for elongation length of greater than or equal to 4 indicates that the mask is not easy to break during the peeling process, and can be removed completely.

Table 1 shows the composition and performance test results of the mask compositions of Examples 1 to 6 of the present invention and Comparative Examples 1 to 2. Table 2 shows the composition and performance test results of the mask compositions of Examples 7 to 11 of the present invention and Comparative Examples 3 to 5. Table 3 shows the composition and performance test results of the mask compositions of Examples 12 to 13 of the present invention and Comparative Examples 6 to 7. Table 4 shows the composition and performance test results of the mask compositions of Examples 14 to 19 of the present invention. Table 5 shows the composition and performance test results of the mask compositions of Examples 20 to 21 of the present invention and Comparative Examples 8 to 9.

**Table 1 Composition and performance test results of the mask compositions of Examples 1 to 6 of the present invention and Comparative Examples 1 to 2**

| Components | Product name | Ex. 1 | Ex. 2 | Ex. 3 | Ex. 4 | Ex. 5 | Ex. 6 | Comp. Ex. 1 | Comp. Ex. 2 |
|---|---|---|---|---|---|---|---|---|---|
| Component a) aqueous polyurethane dispersion | Baycusan® C 1004/1 | 35.0 | 35.0 | 35.0 | 35.0 | 35.0 | 35.0 | 35.0 | 35.0 |
| Thickener different from component b) | Carbopol® Ultrez 20 | 0.2 | 0.2 | 0.2 | 0.2 | 0.2 | 0.2 | 0.2 | 0.2 |
| Component b) liquid inverse emulsion thickener | Simugel INS 100 | 1.5 | - | - | - | - | - | - | - |
| | Sepigel 305 | - | 1.5 | - | - | - | - | - | - |
| | Simugel EG | - | - | 1.5 | - | - | - | - | - |
| | Sepiplus 400 | - | - | - | 1.5 | 0.8 | 6.0 | 0.6 | 6.4 |
| Component c) non-silicone based fat or oil | Caprylic/capric triglyceride | 3.0 | 3.0 | 3.0 | 3.0 | 3.0 | 3.0 | 3.0 | 3.0 |
| Component d) additives | Glycerol | 4.0 | 4.0 | 4.0 | 4.0 | 4.0 | 4.0 | 4.0 | 4.0 |
| | Butanediol | 2.0 | 2.0 | 2.0 | 2.0 | 2.0 | 2.0 | 2.0 | 2.0 |
| | Ethanol | 3.0 | 3.0 | 3.0 | 3.0 | 3.0 | 3.0 | 3.0 | 3.0 |
| | Triethanol amine (99%) | 0.2 | 0.2 | 0.2 | 0.2 | 0.2 | 0.2 | 0.2 | 0.2 |
| | Euxyl®PE 9010 | 0.8 | 0.8 | 0.8 | 0.8 | 0.8 | 0.8 | 0.8 | 0.8 |
| Cosmetic acceptable medium | Deionized water | Up to 100 | Up to 100 | Up to 100 | Up to 100 | Up to 100 | Up to 100 | Up to 100 | Up to 100 |

| Performance Testing | | | | | | | | | |
|---|---|---|---|---|---|---|---|---|---|
| Adhesion | | 5 | 5 | 5 | 5 | 5 | 5 | 5 | 5 |
| Pain feeling | | 5 | 5 | 5 | 5 | 5 | 4 | 5 | 4 |
| Streched length | | 5 | 5 | 5 | 5 | 4 | 4 | 1 | 1 |
| Residual amount of fat or oil | | 5 | 5 | 5 | 5 | 5 | 5 | 5 | 5 |
| Notes | | | | | | | | The sample shrank easily on the skin and could not be spread uniformly | The sample had a too high viscosity and could not be spread easily on the skin surface uniformly |

| | | | | | | | | | |
|---|---|---|---|---|---|---|---|---|---|
| Notes: Unit of component weight: g; the above weight values refer to those including the weight of carriers. | | | | | | | | | |

In Examples 1 to 6, different components b) liquid inverse emulsion thickeners were selected. The samples in the examples were evaluated for adhesion, pain feeling, elongation length, and residual amount of fat or oil, which all showed a score not lower than 4. It was indicated that the mask compositions were removed completely by dry peel-off, which means a good dry peel-off effect, and the users had no obvious greasy feeling. The contents of Sepiplus 400 in Comparative Examples 1 and 2 were 0.6 wt% and 6.4 wt% respectively. The corresponding mask compositions containing said contents of Sepiplus 400 were evaluated for elongation length, which showed a score of 1 and was unqualified. The sample of Comparative Example 1 shrank easily on the skin and could not be spread uniformly. The sample of Comparative Example 2 had a too high viscosity and could not be spread easily on the skin surface uniformly.

**Table 2 Composition and performance test results of the mask compositions of Examples 7 to 11 of the present invention and Comparative Examples 3 to 5**

| Components | Product name | Ex. 7 | Ex. 8 | Ex. 9 | Ex. 10 | Ex. 11 | Comp. Ex. 3 | Comp. Ex. 4 | Comp. Ex. 5 |
|---|---|---|---|---|---|---|---|---|---|
| Component a) aqueous polyurethane dispersion | Baycusan® C 1004/1 | 35.0 | 35.0 | 35.0 | 35.0 | 35.0 | 35.0 | 35.0 | 35.0 |
| Thickener different from component b) | Carbopol® Ultrez 20 | 0.2 | 0.2 | 0.2 | 0.2 | 0.2 | 0.2 | 0.2 | 0.2 |
| Component b) liquid inverse emulsion thickener | Sepiplus 400 | 1.5 | 1.5 | 1.5 | 1.5 | 1.5 | 1.5 | 1.5 | 1.5 |
| fat or oil | Macadamia oil | 3.0 | - | - | - | - | - | - | - |
| | Isododecane | - | 3.0 | - | - | - | - | - | - |
| | Jojoba oil | - | - | 3.0 | - | - | - | - | - |
| | Sweet almond oil | - | - | - | 3.0 | - | - | - | - |
| | Olive oil | - | - | - | - | 3.0 | - | - | - |
| | Dimethylsiloxan e (viscosity = 50cps) | - | - | - | - | - | 3.0 | - | - |
| | DOWSIL™ SH 245 Fluid | - | - | - | - | - | - | 3.0 | - |
| | DOWSIL™ SH 556 Fluid | - | - | - | - | - | - | - | 3.0 |
| Component d) additives | Glycerol | 4.0 | 4.0 | 4.0 | 4.0 | 4.0 | 4.0 | 4.0 | 4.0 |
| | Butanediol | 2.0 | 2.0 | 2.0 | 2.0 | 2.0 | 2.0 | 2.0 | 2.0 |
| | Ethanol | 3.0 | 3.0 | 3.0 | 3.0 | 3.0 | 3.0 | 3.0 | 3.0 |
| | Triethanol amine (99%) | 0.2 | 0.2 | 0.2 | 0.2 | 0.2 | 0.2 | 0.2 | 0.2 |
| | Euxyl®PE 9010 | 0.8 | 0.8 | 0.8 | 0.8 | 0.8 | 0.8 | 0.8 | 0.8 |
| Cosmetic acceptable medium | Deionized water | Up to 100 | Up to 100 | Up to 100 | Up to 100 | Up to 100 | Up to 100 | Up to 100 | Up to 100 |

| Performance Testing | | | | | | | | | |
|---|---|---|---|---|---|---|---|---|---|
| Adhesion | | 4 | 4 | 4 | 4 | 4 | 5 | 5 | 3 |
| Pain feeling | | 4 | 4 | 4 | 4 | 4 | 1 | 3 | 4 |
| Elongation length | | 5 | 5 | 5 | 5 | 5 | 3 | 4 | 5 |
| Residual amount of fat or oil | | 5 | 5 | 5 | 5 | 5 | 5 | 5 | 5 |

| | | | | | | | | | |
|---|---|---|---|---|---|---|---|---|---|
| Notes: Unit of component weight: g; the above weight values refer to those including the weight of carriers. | | | | | | | | | |

In Examples 7 to 11, component C) non-silicone oil based fats or oils different from those in Examples 1 to 6 were selected. The examples were evaluated for adhesion, pain feeling, elongation length, and residual amount of fat or oil, which all showed a score not lower than 4. It was indicated that the mask compositions were removed completely by dry peel-off, which means a good dry peel-off effect, and the users had no obvious greasy feeling. Comparative Examples 3 to 5 comprised silicone oil based fat or oil instead of component c) used in the examples of the present invention. The test results showed that the samples of Comparative Examples 3 to 5 could not take comprehensive account of adhesion, pain peeling, elongation length and residual amount of fat or oil.

**Table 3 Composition and performance test results of the mask compositions of Examples 12 to 13 of the present invention and Comparative Examples 6 to 7**

| Components | Product name | Ex. 12 | Ex. 13 | Comp. Ex. 6 | Comp. Ex. 7 |
|---|---|---|---|---|---|
| Component a) aqueous polyurethane dispersion | Baycusan® C 1004/1 | 35.0 | 35.0 | 35.0 | 35.0 |
| Thickener different from component b) | Carbopol® Ultrez 20 | 0.2 | 0.2 | 0.2 | 0.2 |
| Component b) liquid inverse emulsion thickener | Sepiplus 400 | 1.5 | 1.5 | 1.5 | 1.5 |
| Component c) non-silicone based fat or oil | Caprylic/capric triglyceride | 1.0 | 8.0 | 0.5 | 12.0 |
| Component d) additives | Glycerol | 4.0 | 4.0 | 4.0 | 4.0 |
| | Butanediol | 2.0 | 2.0 | 2.0 | 2.0 |
| | Ethanol | 3.0 | 3.0 | 3.0 | 3.0 |
| | Triethanol amine (99%) | 0.2 | 0.2 | 0.2 | 0.2 |
| | Euxyl®PE 9010 | 0.8 | 0.8 | 0.8 | 0.8 |
| Cosmetic acceptable medium | Deionized water | Up to 100 | Up to 100 | Up to 100 | Up to 100 |

| Performance Testing | | | | | |
|---|---|---|---|---|---|
| Adhesion | | 5 | 4 | 4 | 4 |
| Pain feeling | | 4 | 5 | 3 | 5 |
| Elongation length | | 5 | 5 | 4 | 5 |
| Residual amount of fat or oil | | 5 | 4 | 5 | 2 |
| Notes | | | | | Too much residue of fat or oil on the skin after |
| | | | | | peel-off, poor skin feel |

| | | | | | |
|---|---|---|---|---|---|
| Notes: Unit of component weight: g; the above weight values refer to those including the weight of carriers. | | | | | |

The examples 12 to 13 were evaluated for adhesion, pain feeling, elongation length, and residual amount of fat or oil, which all showed a score not lower than 4. It was indicated that the mask compositions were removed completely by dry peel-off, which means a good dry peel-off effect, and the users had no obvious greasy feeling. Comparative Example 6 comprised 0.5 wt% of caprylic/capric triglyceride, while Comparative Example 7 comprised 12 wt% of caprylic/capric triglyceride. The test results showed that the samples of Comparative Examples 6 to 7 could not take comprehensive account of adhesion, pain peeling, elongation length and residual amount of fat or oil.

**Table 4 Composition and performance test results of the mask compositions of Examples 14 to 19 of the present invention**

| Components | Product name | Ex. 14 | Ex. 15 | Ex. 16 | Ex. 17 | Ex. 18 | Ex. 19 |
|---|---|---|---|---|---|---|---|
| Component a) aqueous polyurethane dispersion | Baycusan® C1001/1 | 35.0 | - | - | - | - | - |
| | Baycusan® C1004/1 | - | 35.0 | - | - | - | - |
| | Baycusan® C1008/1 | - | - | 35.0 | - | - | - |
| | Carfil®ST11 | - | - | - | 35.0 | - | - |
| | Baycusan® C1004 | - | - | - | - | 35.0 | - |
| | Carfil® 9221 | - | - | - | - | - | 35.0 |
| Thickener different from component b) | Carbopol® Ultrez 20 | 0.2 | 0.2 | 0.2 | 0.2 | 0.2 | 0.2 |
| Component b) liquid inverse emulsion thickener | Sepiplus 400 | 1.5 | 1.5 | 1.5 | 1.5 | 1.5 | 1.5 |
| Component c) non-silicone based fat or oil | Caprylic/capric triglyceride | 1.0 | 1.0 | 1.0 | 1.0 | 1.0 | 1.0 |
| Component d) additives | Glycerol | 4.0 | 4.0 | 4.0 | 4.0 | 4.0 | 4.0 |
| | Butanediol | 2.0 | 2.0 | 2.0 | 2.0 | 2.0 | 2.0 |
| | Ethanol | 3.0 | 3.0 | 3.0 | 3.0 | 3.0 | 3.0 |
| | Triethanol amine (99%) | 0.2 | 0.2 | 0.2 | 0.2 | 0.2 | 0.2 |
| | Dermosoft OMP | 1.0 | 1.0 | 1.0 | 1.0 | 1.0 | 1.0 |
| | Sensocel 200 (original wood) | 2.0 | 2.0 | 2.0 | 2.0 | 2.0 | 2.0 |
| Cosmetic acceptable medium | Deionized water | Up to 100 | Up to 100 | Up to 100 | Up to 100 | Up to 100 | Up to 100 |

| Performance Testing | | | | | | | |
|---|---|---|---|---|---|---|---|
| Adhesion | | 5 | 5 | 5 | 4 | 5 | 5 |
| Pain feeling | | 5 | 5 | 4 | 5 | 5 | 4 |
| Elongation length | | 5 | 5 | 5 | 5 | 5 | 5 |
| Residual amount of fat or oil | | 5 | 5 | 5 | 5 | 5 | 5 |

| | | | | | | | |
|---|---|---|---|---|---|---|---|
| Notes: Unit of component weight: g; the above weight values refer to those including the weight of carriers. | | | | | | | |

The samples of Examples 14 to 19 comprised different aqueous polyurethane dispersions. The score for the adhesion, pain feeling, elongation length, and residual amount of fat or oil were all not lower than 4. It was indicated that the mask composition was removed completely by dry peel-off, which means a good dry peel-off effect, and the users had no obvious greasy feeling.

**Table 5 Composition and performance test results of the mask compositions of Examples 20 to 21 of the present invention and Comparative Examples 8 to 9**

| Components | Product name | Ex. 20 | Ex. 21 | Comp. Ex. 8 | Comp. Ex. 9 |
|---|---|---|---|---|---|
| Component a) aqueous polyurethane dispersion | Baycusan® C1004/1 | 20.0 | 70.0 | 75.0 | 15.0 |
| Thickener different from component b) | Carbopol® Ultrez 20 | 0.2 | 0.2 | 0.2 | 0.2 |
| Component b) liquid inverse emulsion thickener | Sepiplus 400 | 1.5 | 1.5 | 1.5 | 1.5 |
| Component c) non-silicone based fat or oil | Caprylic/capric triglyceride | 1.0 | 1.0 | 1.0 | 1.0 |
| Component d) additives | Glycerol | 4.0 | 4.0 | 4.0 | 4.0 |
| | Butanediol | 2.0 | 2.0 | 2.0 | 2.0 |
| | Ethanol | 3.0 | 3.0 | 3.0 | 3.0 |
| | Triethanol amine (99%) | 0.2 | 0.2 | 0.2 | 0.2 |
| | Euxyl®PE 9010 | 0.8 | 0.8 | 0.8 | 0.8 |
| Cosmetic acceptable medium | Deionized water | Up to 100 | Up to 100 | Up to 100 | Up to 100 |

| Performance Testing | | | | | |
|---|---|---|---|---|---|
| Adhesion | | 5 | 4 | 3 | 1 |
| Pain feeling | | 5 | 5 | 4 | 5 |
| Elongation length | | 5 | 5 | 5 | 5 |
| Residual amount of fat or oil | | 5 | 5 | 5 | 5 |
| Notes | | | | | The film broke easily during peel-off |

| | | | | | |
|---|---|---|---|---|---|
| Notes: Unit of component weight: g; the above weight values refer to those including the weight of carriers. | | | | | |

The score of the samples of Examples 20 to 21 for the adhesion, pain feeling, elongation length, and residual amount of fat or oil were all not lower than 4. It was indicated that the mask compositions were removed completely by dry peel-off, which means a good dry peel-off effect, and the users had no obvious greasy feeling. The amount of polyurethane in the mask compositions of Comparative Example 7 and Comparative Example 8 was less than 8 wt% or more than 29 wt%. The test results showed that the samples of Comparative Example 8 and Comparative Example 9 could not take comprehensive account of adhesion, pain peeling, elongation length and residual amount of fat or oil.

Those skilled in the art will readily understand that the present invention is not limited to the foregoing specific details. The present invention can be implemented in other specific forms without departing from the spirit or main characteristics of the present invention. The embodiments should therefore be considered as illustrative and not restrictive from any point of view. The scope of the present invention is indicated by the claims rather than by the foregoing description. Therefore, any change should be regarded as belonging to the present invention, as long as it is within the scope of equivalents of the claims.

## Claims

1. A peel-off mask composition, comprising the following components:
a) at least an aqueous polyurethane dispersion comprising a polyurethane and water;
b) at least a liquid inverse emulsion thickener;
c) at least a non-silicone oil based fat or oil, and
d) optionally an additive;
wherein the amount of the polyurethane is 8 wt% to 29 wt%, the amount of the liquid inverse emulsion thickener is 0.8 wt% to 6 wt%, the amount of the non-silicone oil based fat or oil is 1 wt% to 8 wt%, the above weight proportions being all based on the amount of the mask composition being 100 wt%.

2. The mask composition according to claim 1, **characterized in that** the solid content of the aqueous polyurethane dispersion is 10 wt% to 15 wt%, based on the amount of the aqueous polyurethane dispersion being 100 wt%.

3. The mask composition according to claim 1 or 2, **characterized in that** the amount of the liquid inverse emulsion thickener is 1 wt% to 3 wt%, based on the amount of the mask composition being 100 wt%.

4. The mask composition according to any one of claims 1 to 3, **characterized in that** the amount of the non-silicone oil based fat or oil is 3 wt% to 5 wt%, based on the amount of the mask composition being 100 wt%.

5. The mask composition according to any one of claims 1 to 4, **characterized in that** the polyurethane is obtained by the reaction of a system comprising a1) at least a water-insoluble and water-nondispersible isocyanate-functionalized polyurethane prepolymer and a2) at least an isocyanate-reactive compound.

6. The mask composition according to any one of claims 1 to 5, **characterized in that** the polyurethane comprises at least a sulfonic acid group and/or sulfonate group.

7. The mask composition according to any one of claims 1 to 6, **characterized in that** the liquid inverse emulsion thickener is a compounded product obtained from a system comprising at least one of alkanes and isoalkanes, a hydrophobically modified acrylic copolymer and an nonionic emulsifier of oil-in-water type, wherein the continuous phase of the liquid inverse emulsion thickener is the at least one of alkanes and isoalkanes, and the internal phase is the hydrophobically modified acrylic copolymer.

8. The mask composition according to claim 7, **characterized in that** the hydrophobically modified acrylic copolymer is pre-neutralized, and most preferably a pre-neutralized hydrophobically modified acrylic copolymer in a spiral form.

9. The mask composition according to claim 7 or 8, **characterized in that** the hydrophobically modified acrylic copolymer is one or more selected from polyacrylamide, sodium acryloyldimethyltaurate copolymer and ammonium acryloyldimethyltaurate copolymer.

10. The mask composition according to any one of claims 1 to 9, **characterized in that** the non-silicone oil based fat or oil is one or more selected from vegetable oils, animal fats, mineral oils, and synthetic fats or oils, most preferably one or more selected from caprylic/capric triglyceride, macadamia oil and isododecane.

11. The mask composition according to any one of claims 1 to 10, **characterized in that** the additive is one or more selected from thickeners different from component b), fats or oils different from component c), emulsifiers, preservatives and humectants.

12. The mask composition according to claim 11, **characterized in that** the amount of the thickener different from component b) is not more than 10 wt%, based on the amount of the mask composition being 100 wt%.

13. The mask composition according to claim 11, **characterized in that** the amount of the fat or oil different from component c) is not more than 10 wt%, based on the amount of the mask composition being 100 wt%.

14. The mask composition according to any one of claims 1 to 13, **characterized in that** the mask composition is a dry peel-off mask.

15. A method for preparing a peel-off mask composition according to any one of claims 1 to 14, including the following steps:
mixing component a) aqueous polyurethane dispersion, component b) liquid inverse emulsion thickener, component c) non-silicone oil based fat or oil and component d) optionally additives to obtain the mask composition.

16. Use of the peel-off mask composition according to any one of claims 1 to 14 for applying on the skin to take care of the skin.

17. A method for taking care of the skin, including:
applying the peel-off mask composition according to any one of claims 1 to 14 on the skin surface;
drying to form a film; and
peeling it from the skin.
